(19) Europäisches Patentamt
European Patent Office
Office européen des brevets

(11) Veröffentlichungsnummer: **0 272 590 B1**

(12) **EUROPÄISCHE PATENTSCHRIFT**

(45) Veröffentlichungstag der Patentschrift: **09.03.94**

(51) Int. Cl.5: **C07D 487/22**, C07D 519/00, C08G 12/26, C08K 5/34, C08L 61/20, //(C07D519/00, 487:00,487:00),(C07D487/22, 251:00,251:00,235:00,235:00)

(21) Anmeldenummer: **87118607.8**

(22) Anmeldetag: **15.12.87**

Die Akte enthält technische Angaben, die nach dem Eingang der Anmeldung eingereicht wurden und die nicht in dieser Patentschrift enthalten sind.

# (54) Glykolurilderivate und ihre Verwendung als Stabilisatoren für Polymere.

(30) Priorität: **22.12.86 DE 3643892**

(43) Veröffentlichungstag der Anmeldung: **29.06.88 Patentblatt 88/26**

(45) Bekanntmachung des Hinweises auf die Patenterteilung: **09.03.94 Patentblatt 94/10**

(84) Benannte Vertragsstaaten: **BE CH DE FR GB IT LI NL**

(56) Entgegenhaltungen:
**EP-A- 0 213 570**
**WO-A-81/01706**
**DE-A- 2 623 143**
**FR-A- 2 291 203**

(73) Patentinhaber: **BASF Aktiengesellschaft**
**Carl-Bosch-Strasse 38**
**D-67063 Ludwigshafen(DE)**

(72) Erfinder: **Aumueller, Alexander, Dr.**
**An der Marlach 5**
**D-6705 Deidesheim(DE)**
Erfinder: **Neumann, Peter, Dr.**
**Franz-Schubert-Strasse 1**
**D-6908 Wiesloch(DE)**
Erfinder: **Trauth, Hubert**
**Milanstrasse 5**
**D-6724 Dudenhofen(DE)**

EP 0 272 590 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß 2,2,6,6-Tetraalkyl-piperidinderivate Lichtschutzmittel für organische Polymere sind. In der FR-A-2 291 203 sind Glykolurilderivate der Formel

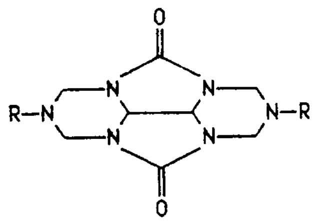

beschrieben, worin R einen gegebenenfalls substituierten Kohlenwasserstoffrest mit bis zu 22 Kohlenstoffatomen bedeutet. Diese Verbindungen werden als oberflachenaktive Mittel für die Textilindustrie und als Korrosionsinhibitoren vorgeschlagen.

Die altere EP-A-213 570 beschreibt Glykolurilderivate mit 2,2,6,6-Tetraalkylpiperidinsubstituenten als Lichtschutzmittel für organische Materialien. Bei diesen Verbindungen ist der Tetraalkylpiperidinrest jeweils direkt an das heterocyclische Ringsystem gebunden.

In den Schriften DE-A-26 36 143 und WO 81/01706 werden 2,2,6,6-Tetramethylpiperidinderivate mit bestimmten Brückengliedern zwischen zwei Polyalkylpiperidinresten als Lichtschutzmittel für Polymere beschrieben.

Unbefriedigend ist oft die Vertraglichkeit dieser Mittel insbesondere mit Polyolefinen, die Dauer der Schutzwirkung. die Neigung zur Flüchtigkeit und die Eigenfarbe der Substanzen.

Der Erfindung liegt die Aufgabe zugrunde, neue Glykolurilderivate zur Verfügung zu stellen, welche sich zum Stabilisieren von organischem Material eignen und die frei von den vorstehenden Nachteilen sind.

Diese Aufgabe wird gelost durch die Verbindungen der allgemeinen Formel (I)

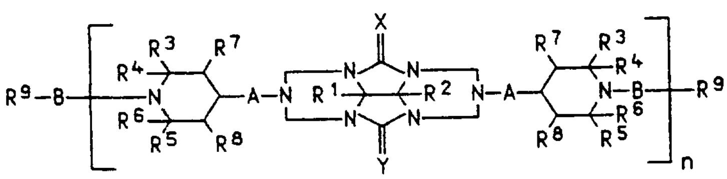

(I)

worin:

n für die Zahl 1 steht,

$R^1$ und $R^2$ unabhängig voneinander Wasserstoff, $C_1$-$C_6$-Alkyl, $C_7$-$C_{12}$-Aralkyl, gegebenenfalls durch $C_1$-$C_4$-Alkyl, $C_1$-$C_4$-Alkoxy oder Halogen substituiertes Phenyl, oder Naphthyl oder eine Carbonestergruppe der Formel

$$-\overset{O}{\overset{\|}{C}}-O-C_1-C_4\text{-Alkyl}$$

darstellen oder $R^1$ und $R^2$ zusammen eine Tetra-, Penta- oder Hexamethylengruppe oder einen Rest der Formel

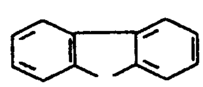

bedeuten,

$R^3$, $R^4$, $R^5$ und $R^6$ unabhängig voneinander für $C_1$-$C_6$-Alkyl stehen,

$R^7$ und $R^8$ unabhängig voneinander für Wasserstoff oder $C_1$-$C_4$-Alkyl stehen oder mit dem zugehörigen Kohlenstoffatom eine

$>$C = O Gruppe bilden,

X und Y unabhängig voneinander die Bedeutungen Sauerstoff, Schwefel oder $NR^{10}$ besitzen, wobei $R^{10}$ Wasserstoff, $C_1$-$C_8$-Alkyl oder $C_7$-$C_{12}$-Aralkyl ist,

$R^9$ für Wasserstoff, $C_1$-$C_{22}$-Alkyl, das durch Hydroxyl, Carbonyl, $C_1$-$C_4$-Alkoxycarbonyl, Carboxyl, Carbamoyl, Sulfonyl, Sulfinyl oder $-CH_2-CH_2-SH$ substituiert sein kann, $C_3$-$C_{22}$-Alkenyl, $C_3$-$C_{22}$-Alkinyl, $C_7$-$C_{12}$-Aralkyl, $C_1$-$C_{22}$-Alkyl, $C_4$-$C_{22}$-Cycloalkylalkyl, einen Heterocyclus der aus der Gruppe

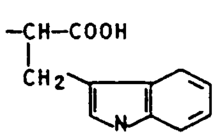 , 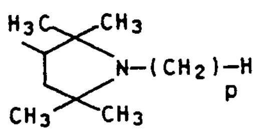 , 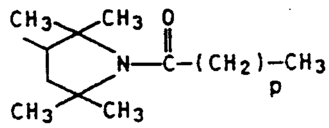 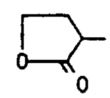 ,

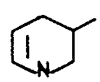 , 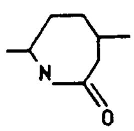 , 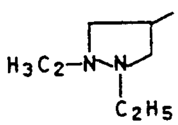 , 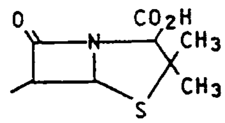 ,

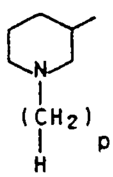 , 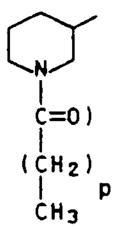 , 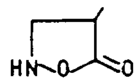 , 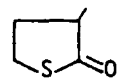 ,

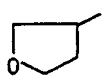 , 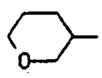 , 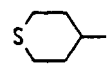 , 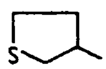 ,

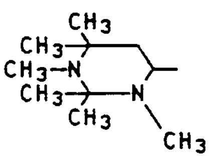 ,

wobei p = 1 bis 20 bedeutet,

Hydroxy, Carboxyl, $C_1$-$C_4$-Alkoxycarbonyl, Sulfonamido, $(R^{13})_2NCO$-, worin $R^{13}$ für Wasserstoff oder $C_1$-$C_4$-Alkyl steht, eine Harnstoffgruppierung der Formel

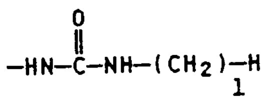 , 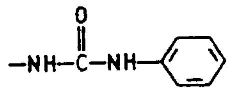 , 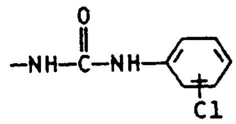 ,

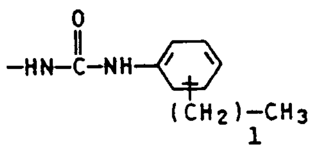 , 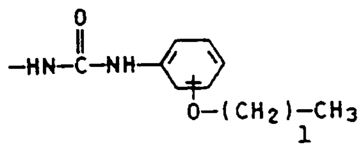 ,

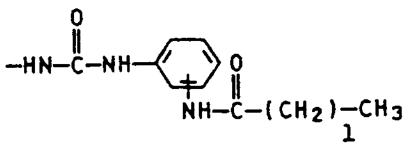 , 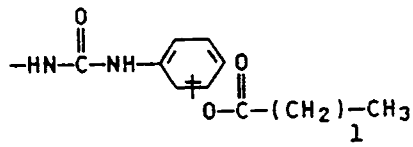 ,

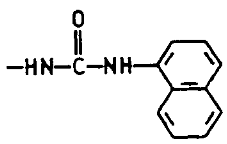 oder 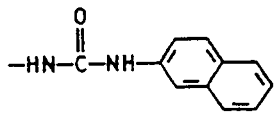 ,

mit l = 0 bis 22,
eine Urethangruppe der Formel

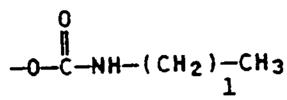

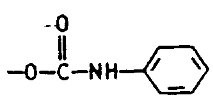 , 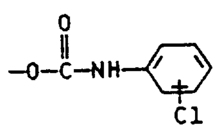 , 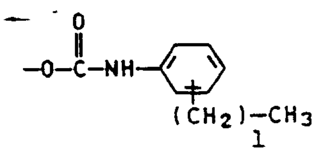

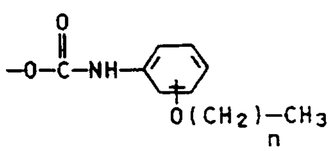 , 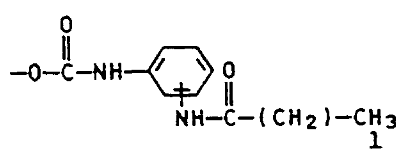 ,

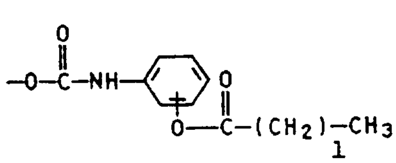 , 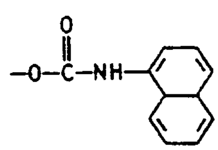 oder

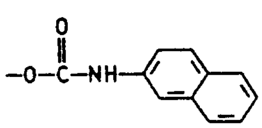 ,

mit l = 0 bis 22,
oder für

$-(CH_2)_k D$

steht, worin k eine Zahl von 1 bis 10 und D -CH, $-NH_2$, $-NHR^{11}$ oder $-NR^{11}R^{12}$ bedeuten, worin $R^{11}$ und $R^{12}$ gleich oder verschieden sind und für $C_1$-$C_{22}$-Alkyl, eine Acylgruppe der Formel

$$-\overset{\overset{\displaystyle O}{\|}}{C}-(CH_2)_l-CH_3$$

mit l = 0 bis 22,
Carbamoyl, Sulfonyl, Sulfinyl, $C_2$-$C_{22}$-Alkenyl, $C_3$-$C_{22}$-Alkinyl, $C_3$-$C_{12}$-Cycloalkyl, oligomeres oder polymeres Polyamin der Formel

$H_2N\{CH_2-CH_2-NH\}_x$ (x = 1 bis 30)

stehen, oder worin $R^{11}$ und $R^{12}$ zusammen mit dem Stickstoffatom, an das sie gebunden sind, ein 3- bis 20-gliedriges Ringsystem bilden,

A gleich oder verschieden ist und eine direkte Bindung oder ein Brückenglied der Formel

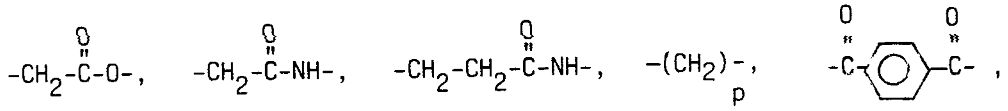

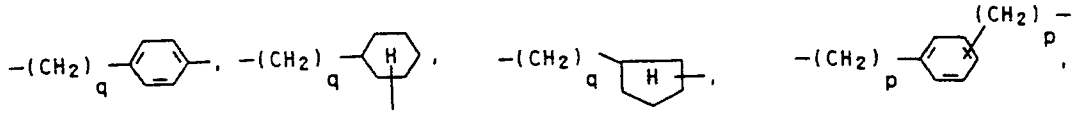

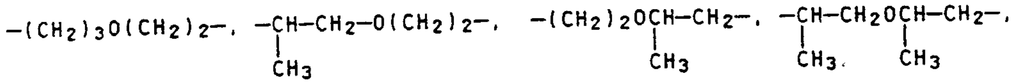

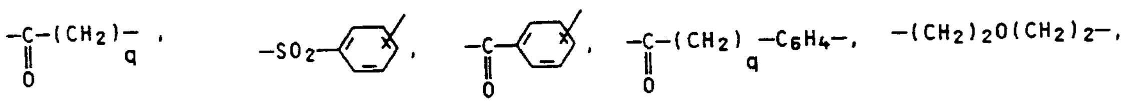

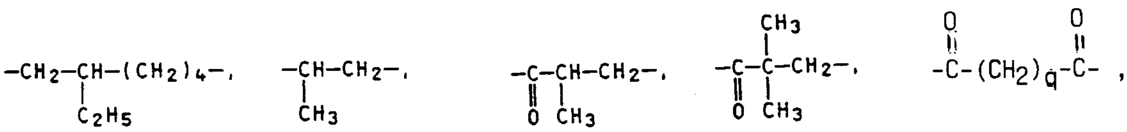

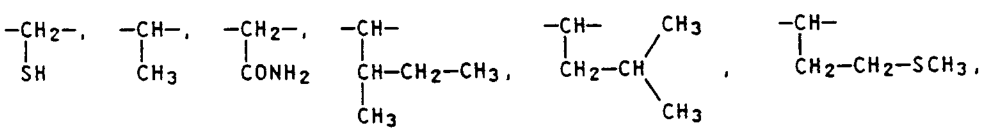

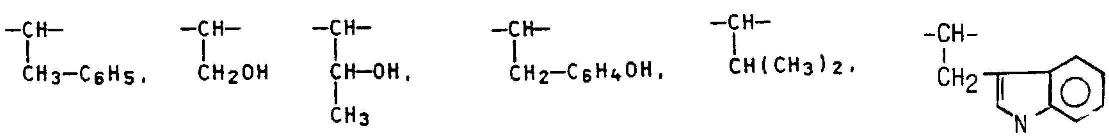

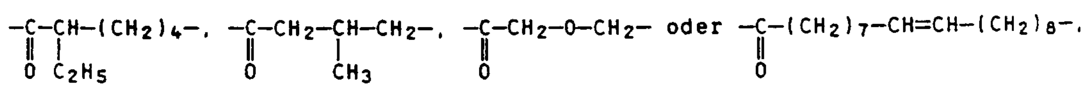
oder

wobei p = 1 bis 20 und q bis zu 4 ist,

darstellt mit der Maßgabe, daß mindestens einer der Reste A für ein Brückenglied steht, falls $R^9$ nicht die Bedeutung $-(CH_2)_k$ D besitzt, und

B eine direkte Bindung darstellt,

und die Säureadditionssalze und Hydrate der Verbindungen der allgemeinen Formel I.

Gegenstand der Erfindung ist auch die Verwendung der Verbindungen der allgemeinen Formel I und deren Gemische zum Stabilisieren von Kunststoffen und Lacken.

Bevorzugt ist die Verwendung der erfindungsgemäßen Verbindungen zum Stabilisieren von Polyolefinen, Polyamiden und Lacken.

Eine besonders bevorzugte Verwendung ist die zum Licht- und Wärmeschutz und als Metallionendesaktivator, insbesondere bei Kunststoffen.

Einzelne Reste $R^1$ und $R^2$ sind neben Wasserstoff z.B.: Methyl, Ethyl, Propyl, Butyl, Pentyl, Hexyl, Benzyl, Phenylethyl, Phenylpropyl, Phenylbutyl, Methylbenzyl, Phenyl, Tolyl, Carbomethoxy, Carboethoxy, Carbopropoxy oder Carbobutoxy.

Bevorzugt sind für $R^1$ und $R^2$ Ethyl, Benzyl, Carbomethoxy oder Carboethoxy und insbesondere Wasserstoff, Methyl oder Phenyl.

$R^9$ steht vorzugsweise für Wasserstoff, $C_1$-$C_{22}$-Alkyl, insbesondere $C_1$-$C_4$-Alkyl, bevorzugt Methyl,

das durch eine Hydroxyl- oder Carboxylgruppe substituiert sein kann, $C_3$-$C_8$-Alkenyl, insbesondere Allyl, $C_7$-$C_{12}$-Aralkyl, insbesondere Benzyl, -$CH_2$-CN, -$(CH_2)_2$-CN; weitere Bedeutungsmöglichkeiten für $R^9$ sind beispielsweise die folgenden:

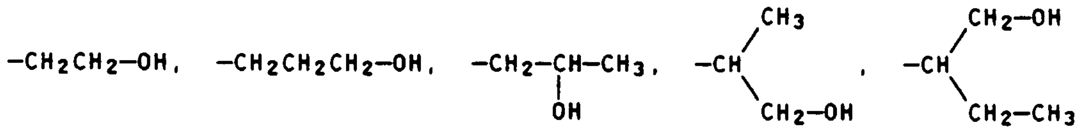

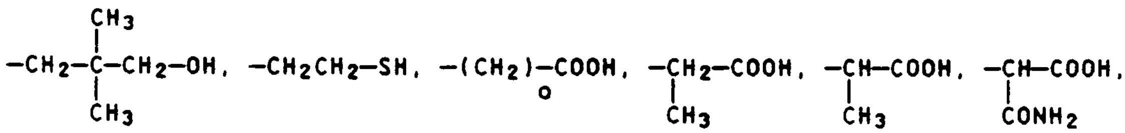

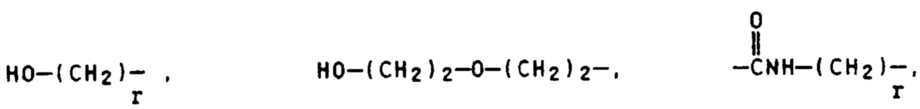

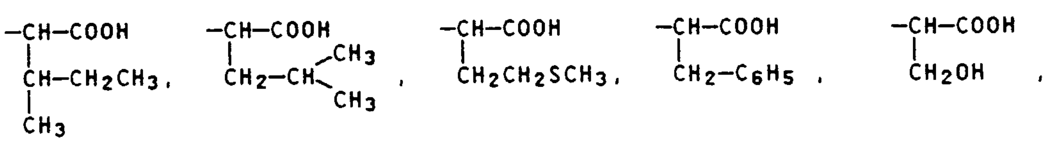

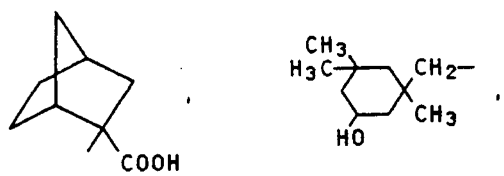

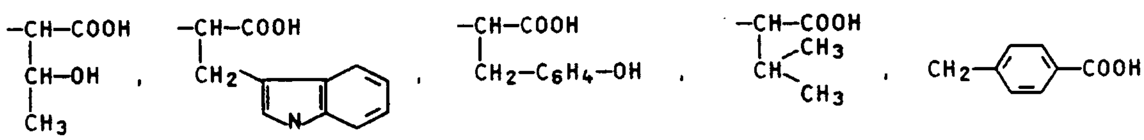

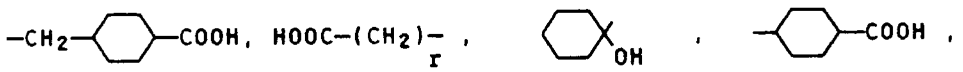

und

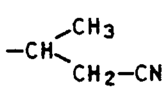

wobei o = 1 bis 21 und r = 1 bis 20 sind.

Die Reste $R^3$, $R^4$, $R^5$ und $R^6$ stehen für $C_1$-$C_6$-Alkylreste, insbesondere für $C_1$-$C_4$-Alkyl, z.B. Methyl, Ethyl, Propyl, Butyl, Pentyl, Hexyl. Besonders bevorzugt sind Methyl und Ethyl.

Der Begriff Aryl steht für Phenyl, dessen $C_1$-$C_4$-Alkyl- oder $C_1$-$C_4$-Alkoxy-Substitutionsprodukte, beispielsweise Tolyl oder Xylyl sowie für Halogenphenyl oder Naphthyl.

Der Begriff $C_3$-$C_{12}$-Cycloalkyl steht vorzugsweise für

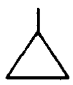 , 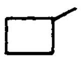 , 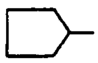 , 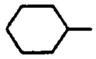 , 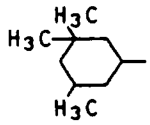 , 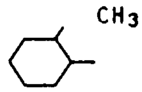 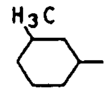

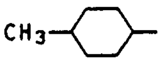 , 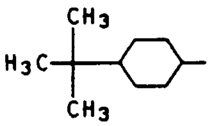 , 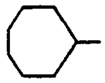 , 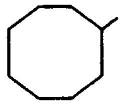 , 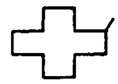 ,

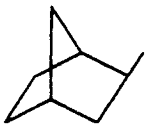 , 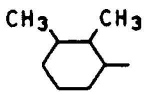 , 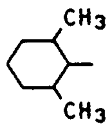 , 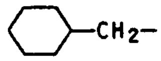 ,

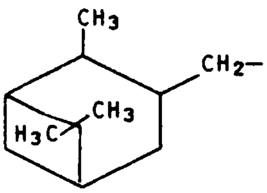 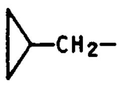 , 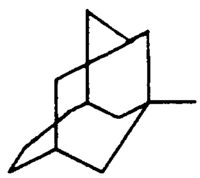

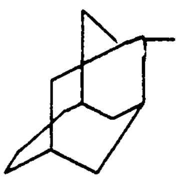 , 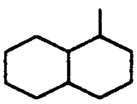 , 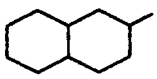 , 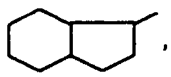 , 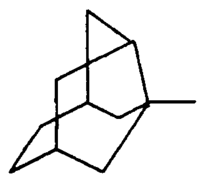

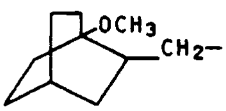 , 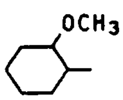 , 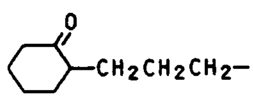 ,

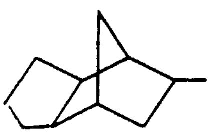 , 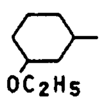 , 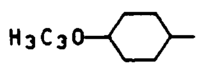 ,

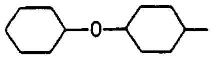 , 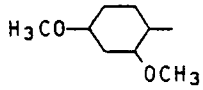 , 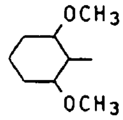 ,

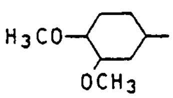 , 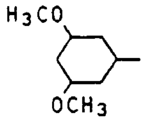 , 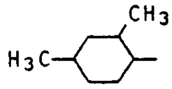 , 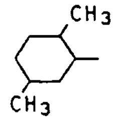

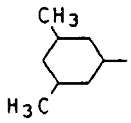 , 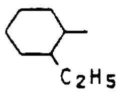 , 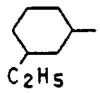 , 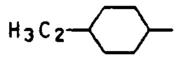 , 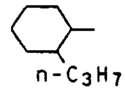 ,

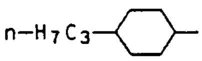 , 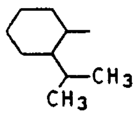 , 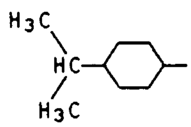 , 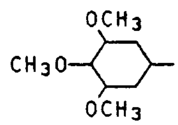 ,

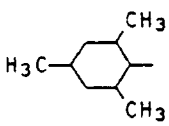 , 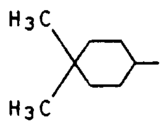 , 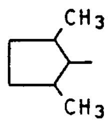 ,

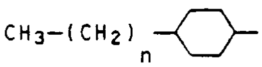 und 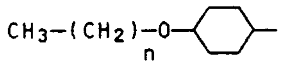 ,

mit n = 1 bis 20.

Die Reste $R^7$ und $R^8$ stehen vorzugsweise für Wasserstoff oder $C_1$-$C_4$-Alkyl, beispielsweise Methyl.

Der hier verwendete Begriff ($C_1$-$C_{22}$-)Alkyl umfaßt geradkettige und verzweigte Reste, insbesondere $C_1$-$C_6$-Alkyl, beispielsweise Methyl, Ethyl. n-Propyl, i-Propyl, Butyl, Pentyl und Hexyl.

Beispiele für andere, verzweigte Alkylreste sind die folgenden:

$(CH_2)_n-CH_3$, 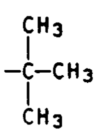 , 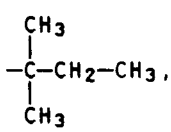 , 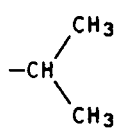 , 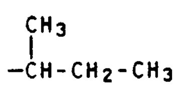 , 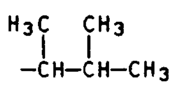 ,

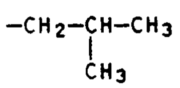 , 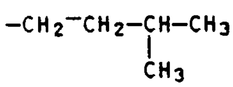 oder 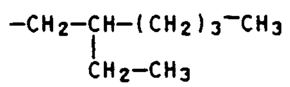 ,

wobei n = 0-21 ist.

Der Begriff $C_7$-$C_{12}$-Aralkyl umfaßt vorzugsweise $C_7$-$C_{12}$-Phenylalkyl wie Benzyl oder Phenylethyl, sowie deren $C_1$-$C_4$-Alkyl- oder $C_1$-$C_4$-Alkoxy-Substitutionsprodukte,

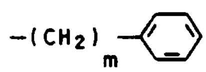 , 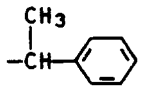 , 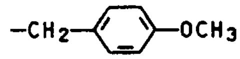 , 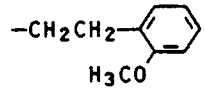

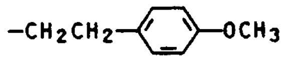 , 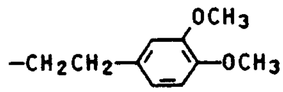 , 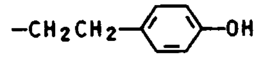 , 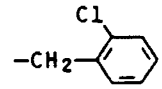 .

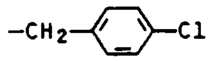 oder 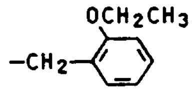 ,

wobei m = 1 bis 6 ist,

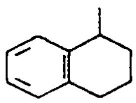 , 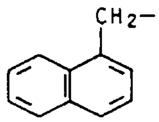 , 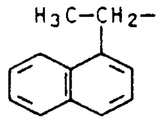 , 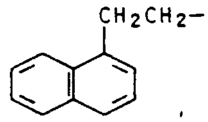 ,

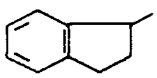 , 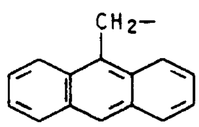 , 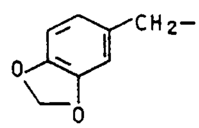 ,

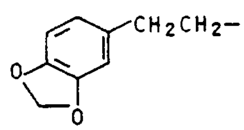 , 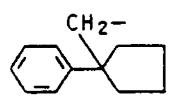 ,

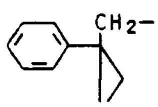 , 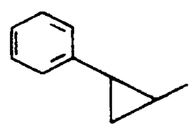 , 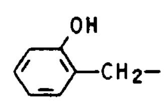 ,

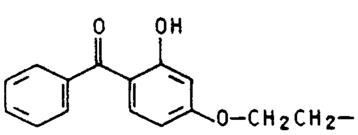 , 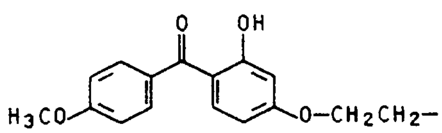 ,

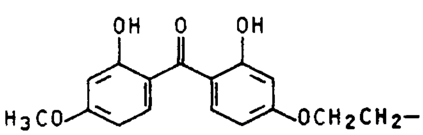 , 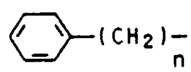 ,

wobei = 1 bis 20 ist.

Der Begriff $C_3$-$C_{22}$-Alkenyl umfaßt geradkettige und verzweigte sowie auch mehrfach ungesättigte Reste. Bevorzugt ist $C_3$-$C_{12}$-Alkenyl, beispielsweise Allyl, Butenyl, Pentenyl, Hexenyl oder Heptenyl.

Der Begriff $C_3$-$C_{22}$-Alkinyl steht für geradkettige oder verzweigte, einfach oder mehrfach ungesättigte Reste. Bevorzugt ist $C_3$-$C_{12}$-Alkinyl, beispielsweise Propargyl, Butinyl, Pentinyl, Hexinyl oder Octinyl.

Bevorzugte Brückenglieder sind $(CH_2)_n$, wobei n insbesondere 1 bis 5, speziell 1, 2, 5 ist.

Y und Z sind unabhängig voneinander insbesondere Sauerstoff, daneben auch Schwefel oder $-NR^{10}$.
$R^{10}$ ist Wasserstoff, $C_1$- bis $C_8$-Alkyl oder $C_7$- bis $C_{12}$-Aralkyl.
$R^{11}$ und $R^{12}$ sind unabhängig voneinander insbesondere Wasserstoff, daneben auch

$-(CH_2)_l-CH_3$, 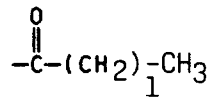 ,

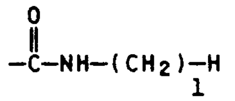 , 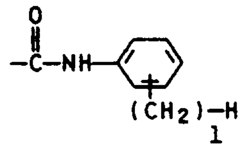 , 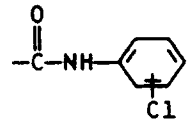 ,

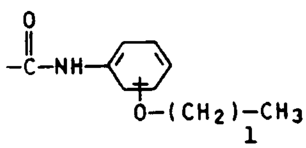 , 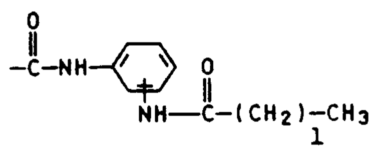 oder 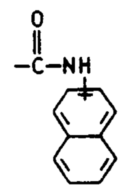 ,

wobei l = 0 bis 22 ist.

Soweit $R^{11}$ und $R^{12}$ zusammen mit dem Stickstoffatom, an das sie gebunden sind, ein Ringsystem bilden, kann dies beispielsweise sein:

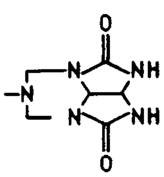 , 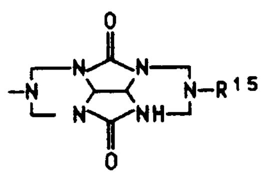 , 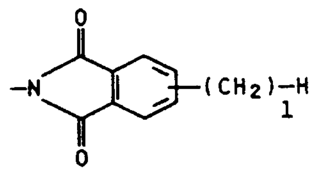 ,

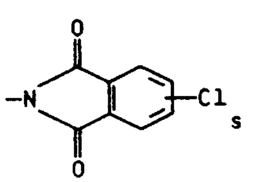 , 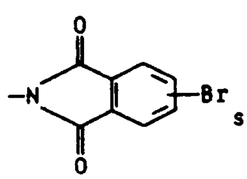 , 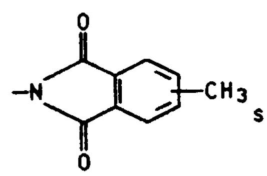 ,

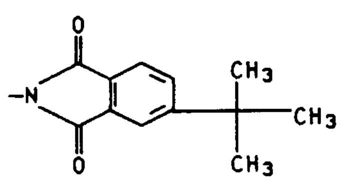 , 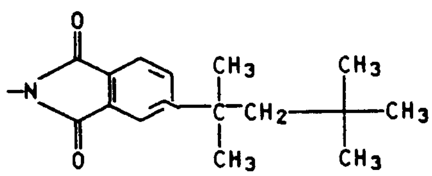 , 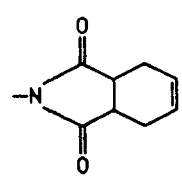

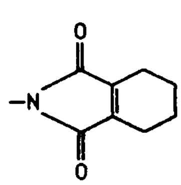 oder 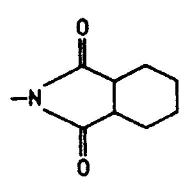 .

wobei l = 0 bis 22 und
s = 1 bis 4 ist,
und $R^{15}$ alle vorstehenden Bedeutungen von $R^9$ annehmen kann.

Gegenstand der vorliegenden Erfindung ist weiterhin ein Produkt erhalten durch die Umsetzung des Amins folgender Struktur:

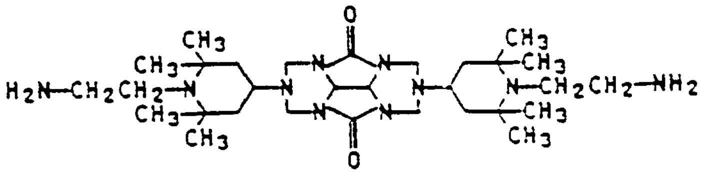

mit Adipinsäurechlorid im Molverhältnis 1:1 in Pyridin, Fällung durch Petrolether und Aufarbeitung mit 10% NaOH, welches folgendes Strukturelement enthält

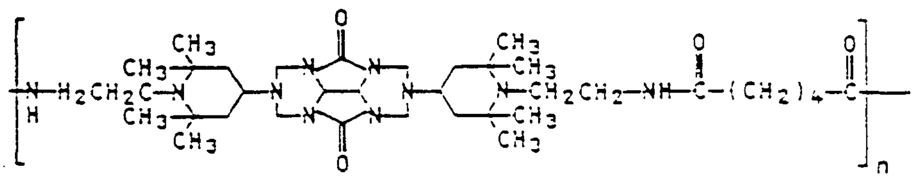

vom Schmelzpunkt 274 °C und einer mittleren Molmasse von 2570 g/mol (dampfdruckosmometrisch in Chloroform). Die Herstellung dieses Produktes wird in Beispiel 11 beschrieben.

Verbindungen der allgemeinen Formel (I) mit n = 1, in denen das Brückenglied A die Gruppierungen -CO-O- oder -CO-NH- aufweist, können durch Reaktion von Verbindungen der allgemeinen Formel II

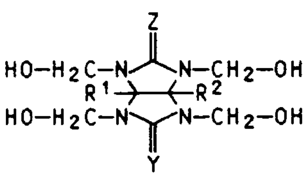

(II)

analog dem Verfahren der FR-A-2 291 203 mit Aminocarbonsäureestern $H_2N\text{-}B\text{-}COOR$ oder Aminosulfonsäuren $H_2\text{-}B\text{-}SO_3$ oder den entsprechenden Alkylestern, beispielsweise den Ethylestern, und anschließender katalysierter Umesterung oder Veresterung mit substituierten Piperidin-4-aminen oder Piperidin-4-olen dargestellt werden. Das folgende Schema zeigt die Reaktionsfolge für eine beispielhafte Verbindung III.

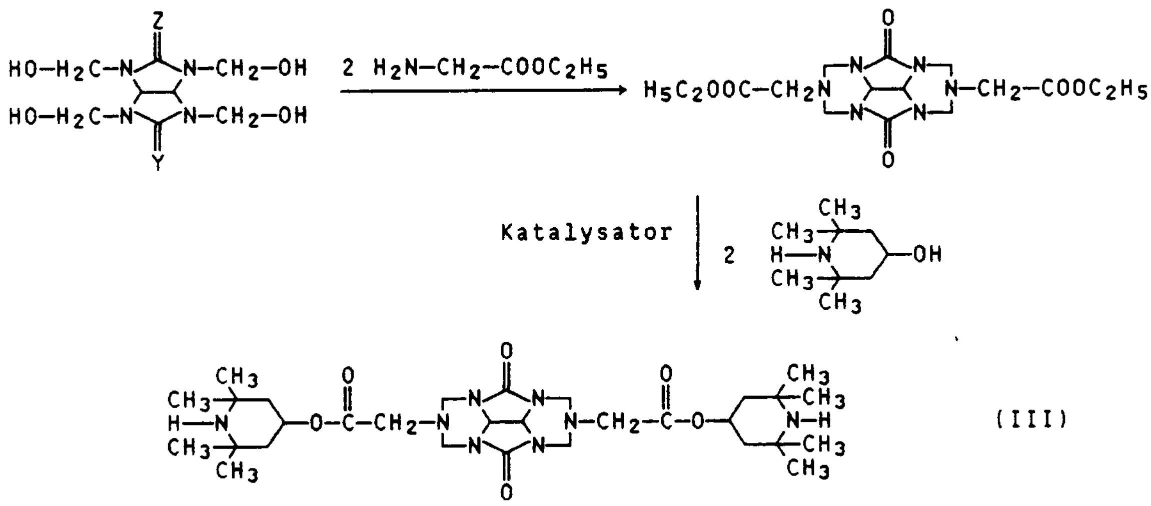


(III)

Der Katalysator des zweiten Reaktionsschrittes kann ein Alkalialkoholat, wie Natriummethylat, ein Alkalihydroxid wie Natriumhydroxid, eine Säure oder bevorzugt ein Tetraalkylorthotitanat, wie Tetrabutylorthotitanat, sein.

Verbindungen der allgemeinen Formel I können weiterhin dargestellt werden durch Umsetzung von Verbindungen der allgemeinen Formel II mit Verbindungen der allgemeinen Formel IV

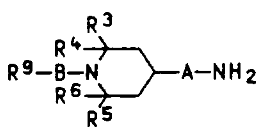

(IV)

Die Verbindungen der Formel II können dabei in situ durch Reaktion von Verbindungen der allgemeinen Formel V

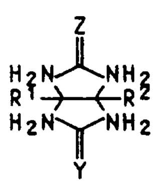

(V)

mit Formaldehyd oder einer Formaldehydquelle hergestellt werden.

Die Verbindungen der Formel I (mit B-$R^9$ = H) können nach literaturbekannten Verfahren, z.B. durch reduktive Aminierung in die Verbindungen mit z.B. B-$R^9$ = $CH_3$ umgewandelt werden.

Verbindungen der allgemeinen Formel I, in denen $R^9$ -$(CH_2)$-CN ist, können in vorteilhafter Weise auch durch Reaktion von Verbindungen der allgemeinen Formel VI

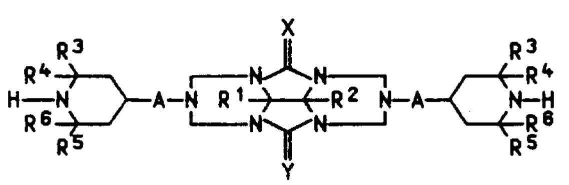

(VI)

mit Glykolsäurenitril oder einer Glykolsäurenitrilquelle hergestellt werden. Die Reaktion von Glykolsäurenitril mit sterisch gehinderten Aminen ist in der DE-A-3 208 570 beschrieben.

Die erfindungsgemäßen Verbindungen können in Form der freien Basen als Hydrate oder als Salze vorliegen. Geeignete Anionen stammen z.B. von anorganischen sauren und insbesondere organischen Carbonsäuren sowie organischen Sulfonsäuren.

Anorganische Anionen sind z.B. Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanonat, Cyclohexanonat, Lactat, Stearat, Dodecylbenzoat, Benzoat, Acrylat, Methacrylat, Citrat, Malonat oder Succinat, sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen besitzen außerordentlich gute stabilisierende Eigenschaften, haben keine Eigenfarbe, sind gut verträglich mit organischen Polymeren und zeigen einen niedrigen Dampfdruck.

Sie eignen sich zum Stabilisieren von organischem Material, speziell von Kunststoffen, gegen den Abbau durch Licht und Warme und gegen schädlichen Einflüsse von Metallen oder Metallionen, die in dem organischen Material in Spuren vorliegen kann. Sie werden den zu stabilisierenden Kunststoffen in einer Konzentration von 0,01 bis 5 Gew.-%, vorzugsweise 0,02 bis 1 Gew.-% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butyl-phenyl)-butan, 1,3,5-Trimetyl-2,4,6-tris-(3,5-di-tert.-butyl-4-hydroxy-benzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxy-ethyl]-isocyanurat, 1,3,5-Tris-(2,6-di-methyl-3-hydroxy-4-tert.-butylbenzyl)-isocyanurat oder Pentaerythrit-tetrakis-[β(3,5-di-tert.-butyl-4-hydroxy-phenyl)-propionat] erwähnt.

Als phosphorhaltige Antioxidantien seien beispielsweise Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit oder Tetrakis-(2,4-ditert.-butylphenyl)-4,4'-biphenylendiphosphit erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) oder Pentaerythrittetrakis-(β-hexylthiopropionat) erwähnt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, α-Cyanozimtsäurederivate. Nickelverbindungen oder Oxalsäuredianilide.

Als organische Polymere, die durch die erfindungsgemäßen Verbindungen stabilisiert werden können, seien beispielsweise genannt:

Polymere von Mono- und Diolefinen, wie z.B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-AcrylsäureCopolymere;

Polystyrol;

Copolymere von Styrol oder α-Methylstyrol mit Dienen oder Acrylderivaten, wie Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie Polyvinylchlorid, Polyvinylfluorid, Polyvinylidenfluorid sowie deren Copolymere;

Polymere, die sich von α,β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen oder deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können, soweit sie fest sind, in fester oder gelöster Form oder, wenn sie flüssig sind, in Substanz dem Lack zugesetzt werden. Ihre gute Löslichkeit in Lacksystemen ist dabei von besonderem Vorteil.

Bevorzugt ist die Verwendung der erfindungsgemäßen Verbindungen in Lacken, Polyamiden oder Polyolefinen, vorzugsweise Ethylen- und Propylenpolymerisaten.

Die Erfindung wird durch die nachstehenden Beispiele weiter erläutert.

Beispiel 1

a) 349 g (= 2,50 Mol) Glycinethylesterhydrochlorid wurden in 380 ml Wasser gelöst. Unter Eiskühlung gab man 100 g festes Natriumhydroxid portionsweise zu. Anschließend wurden 656 g (= 1,25 Mol) einer 50 %igen wäßrigen Lösung von Tetramethylolacetylendiharnstoff zugegeben. Man hielt 2 h am Rückfluß, ließ abkühlen, saugte ab, wusch mit wenig Wasser nach und trocknete im Wasserstrahlvakuum bei 80 °C.
Man isolierte 199 g (40 %) farblosen Feststoff vom Schmp.: 164 °C.
b) 13,6 g Tetracyclo-[5,5,2,$0^{5,13}$,$0^{11,14}$]-1,3,5,7,9,11-hexaaza-3,9-bis-[carbethoxymethyl]-6,12-dioxo-tetradecan aus (a), 10,9 g 4-Hydroxy-2,2,6,6-tetramethylpiperidin und 1,5 ml Tetrabutylorthotitanat wurden unter Stickstoff in 50 ml Xylol 10 h am Rückfluß gekocht. Nach dem Abkühlen wurde der ausgefallene Niederschlag abgesaugt, mit Xylol gewaschen, getrocknet und mit heißem Wasser behandelt. Man erhielt 13,0 g Monohydrat als farblose Kristalle vom Schmp. 183 °C.

| | | | | |
|---|---|---|---|---|
| Berechnet: | C 56,8 | H 7,9 | N 17,6 | O 17,6 |
| gefunden: | C 56,4 | H 8,2 | N 17,6 | O 17,9 |

Beispiel 2

41,4 g des Produkts von Beispiel 1a, 35,7 g 4-Hydroxy-1,2,2,6,6-pentamethylpiperidin und 4,5 ml Tetrabutylorthotitanat wurden in 150 ml Xylol 12,5 h unter Stickstoff am Rückfluß gekocht. Der ausgefallene Niederschlag wurde bei Raumtemperatur abgesaugt, mit Xylol gewaschen und aus Isopropanol umkristallisiert. Man erhielt 18,6 g Monohydrat als farblose Kristalle vom Schmp. 204 °C.

| | | | | |
|---|---|---|---|---|
| Berechnet: | C 58,0 | H 8,5 | N 16.9 | O 16,9 |
| gefunden: | C 58,1 | H 8,3 | N 17,3 | O 16,0 |

Beispiel 3

59,1 g des Produkts von Beispiel 1a, 46,8 g 4-Amino-,2,2,6,6-tetramethylpiperidin 6 ml Tetrabutylorthotitanat wurden in 225 ml Xylol 8 h am Rückfluß gekocht. Durch Aufarbeitung wie in Beispiel 2 erhielt man 23,3 g Tetrahydrat als farblose Kristalle vom Schmp. 201 °C. Nach Einengen des Lösungsmittels auf 250 ml konnten weitere 18,6 g vom Schmp. 199 °C isoliert werden.

| | | | | |
|---|---|---|---|---|
| Berechnet: | C 52,3 | H 8,8 | N 20,3 | O 18,6 |
| gefunden: | C 52,0 | H 8,6 | N 20,3 | O 19,5 |

Beispiel 4

41 g (0,083 Mol) Tetracyclo-[5,5,2,$0^{5.13}$, $0^{11.14}$]-1,3,5,7,9,11-hexaaza-6,12-dioxo-3,9-di(2,2,6,6-tetramethyl-4-piperidinyl)-tetradecan und 20,3 g (0,25 Mol) einer 70 %igen wäßrigen Lösung von Hydroxyessigsäure nitril wurden in 125 ml Ethanol 15 h gekocht. Man ließ abkühlen, saugte ab, wusch mit Ethanol nach bis der Ablauf farblos war und trocknete im Wasserstrahlvakuum bei 60 ° C. Man isolierte 38 g (79 %) farblose Kristalle vom Schmelzpunkt 283 ° C.

| | | | | |
|---|---|---|---|---|
| Ber.: | C 62,1 | H 8,3 | N 24,1 | O 5,6 |
| Gef.: | C 62,1 | H 8,5 | N 24,1 | O 5,6 |

Beispiel 5

30 g (1,0 Mol) Paraformaldehyd, 85 g (1,0 Mol) Acetoncyanhydrin und 2,4 g Kaliumcarbonat wurden in 24 ml mit Kaliumcarbonat gesättigtem Ethanol 2 Stunden bei Raumtemperatur gerührt. Man stellte mit Phosphorsäure auf pH 6 und gab 24 ml 0,5 %ige Phosphorsäure und 350 ml Ethanol zu.

Anschließend wurden 125,5 g (0,25 Mol) Tetracyclo-[5,5,2,$0^{5.13}$,$0^{11.14}$]-1,3,5,7,9,11-hexaaza-6,12-dioxo-3,9-di(2,2,6,6-tetramethyl-4-piperidinyl)-tetradecan zugegeben und die Mischung dann 5 Stunden am Rückfluß erhitzt. Das Produkt wurde wie in Beispiel 4 aufgearbeitet. Man isolierte 123 g (85 % der Theorie) des Produktes von Beispiel 4.

Beispiele 6 bis 9

Analog zu Beispiel 5 werden die Verbindungen

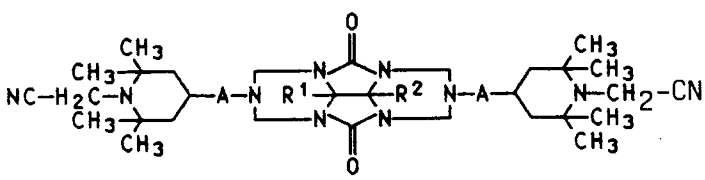

mit den in der nachfolgenden Tabelle angegebenen Resten und Schmelzpunkten hergestellt.

Tabelle:

| Beispiel | $R^1$ | $R^2$ | A | Schmelzpunkt [°C] |
|---|---|---|---|---|
| 6 | $CH_3$ | H | - | 288 |
| 7 | $C_6H_5$ | $C_6H_5$ | - | 308-309 |
| 8 | H | H | $-CH_2\overset{\overset{O}{\|}}{C}O-$ | 202-203 |
| 9 | H | H | $-CH_2\overset{\overset{O}{\|}}{C}NH-$ | 280 |

Beispiel 10

40 g des Produktes aus Beispiel 4 und 5 g Raney-Nickel wurden in einem 1-l Hubautoklav in 500 ml Toluol suspendiert. Es wurden 40 g Ammoniak einkondensiert, mit Wasserstoff wurde ein Druck von 100 bar eingestellt und auf 80 ° C erhitzt. Bei einem Druck von 300 bar wurde hydriert bis keine Wasserstoffaufnahme mehr zu beobachten war.

Zwei solcher Ansätze wurden vereinigt, filtriert und eingeengt. Der Rückstand wurde aus Toluol umkristallisiert, bei 80 ° C getrocknet und mit Wasser ausgekocht.

Ausbeute: 39,6 g des Hydrates der Verbindung der Formel

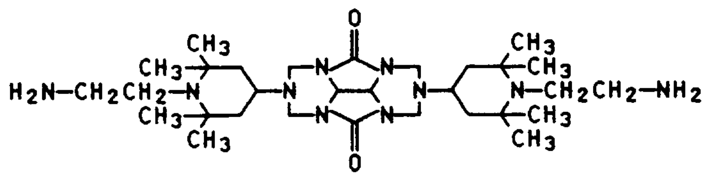

vom Schmelzpunkt 203-204 ° C.

Beispiel 11

Zu 7,7 g des Produktes aus Beispiel 10 in 75 ml Pyridin wurden 2,4 g Adipinsäurechlorid getropft. Die Mischung wurde 3,5 h auf 50-60 ° C gehalten, abgekühlt und auf 150 ml Petrolether gegossen. Der Rückstand wurde abgesaugt, 10 Minuten mit 10 %iger Natronlauge verrührt, abgesaugt und mit Wasser gewaschen. Nach dem Trocknen wurde in wenig Isobutanol heiß gelöst, filtriert, das Filtrat in Petrolether eingerührt, der ausgefallene Niederschlag abgesaugt. Ausbeute: 2,6 g Oligomeres der Formel

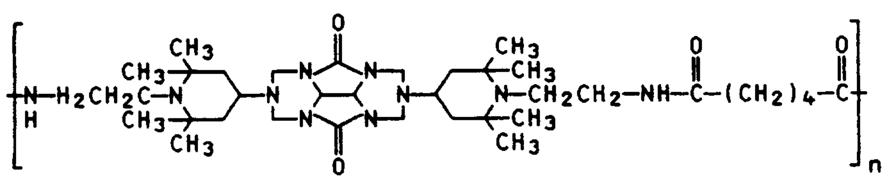

vom Schmelzpunkt 274 ° C. Die mittlere Molmasse beträgt 2570 g/mol (dampfdruckosmometrisch in Chloroform).

Beispiel 12

a) 169,5 g Cyanessigsäureethylester und 232,5 g 2,2,6,6-Tetramethyl-4-amino-piperidin wurden in 200 ml Ethanol 8 h gekocht. Nach dem Abkühlen wurden 5 g Aktivkohle und 450 ml Essigsäureethylester zugegeben, die Mischung wurde erhitzt und heiß filtriert. Nach dem Abkühlen wurden 169,2 g der Verbindung der Formel

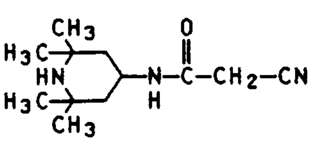

vom Schmelzpunkt 150-152 ° C abgesaugt.

b) 44 g des Produktes aus a) wurden in 400 ml Methanol unter Zusatz von 10 g Raney-Nickel und 40 g Ammoniak bei 200 bar bis zur Druckonstanz hydriert. Die Reaktionsmischung wurde filtriert, eingeengt und einer Vakuumdestillation unterworfen. Man isolierte 21,5 g der Verbindung der Formel

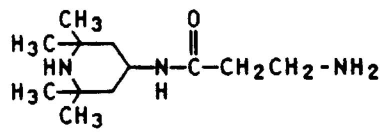

vom Siedepunkt 165-169 °C bei 0,5 Torr. Nach längerem Stehenlassen kristallisierte das erhaltene Öl durch und schmolz bei 74-76 °C.

c) 11,3 g des Produktes aus b) und 13,1 g einer 50 %igen wäßrigen Lösung von Tetramethylolacetylendiharnstoff wurden in 300 ml Isobutanol am Wasserabscheider gekocht bis kein Wasser mehr abgeschieden wurde. Die Lösung wurde eingeengt, der Rückstand aus Acetonitril umkristallisiert. Ausbeute: 7,1 g des Hydrats der Verbindung der Formel

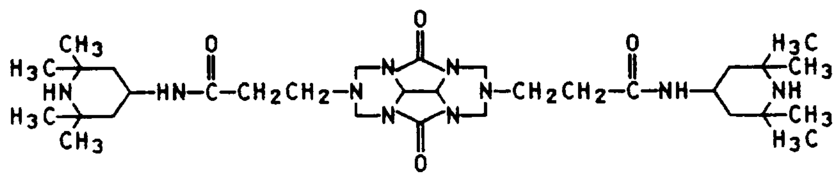

vom Schmelzpunkt 125-127 °C.

**Patentansprüche**

**1.** Verbindungen der allgemeinen Formel (I)

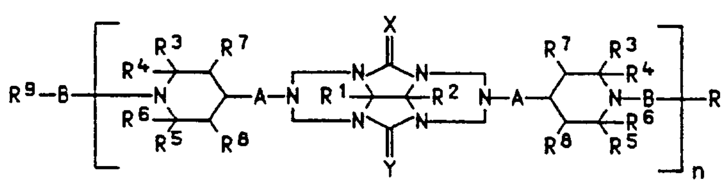

(I),

in der

n für die Zahl 1 steht,

$R^1$ und $R^2$ unabhängig voneinander Wasserstoff, $C_1$-$C_6$-Alkyl, $C_7$-$C_{12}$-Aralkyl, gegebenenfalls durch $C_1$-$C_4$-Alkyl, $C_1$-$C_4$-Alkoxy oder Halogen substituiertes Phenyl, oder Naphthyl oder eine Carbonestergruppe der Formel

$$-\overset{O}{\overset{\|}{C}}-O-C_1-C_4\text{-Alkyl}$$

oder $R^1$ und $R^2$ zusammen eine Tetra-, Penta- oder Hexamethylengruppe oder einen Rest der Formel

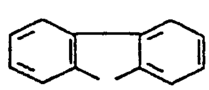

bedeuten,

$R^3$, $R^4$, $R^5$ und $R^6$ unabhängig voneinander für $C_1$-$C_6$-Alkyl stehen,

$R^7$ und $R^8$ unabhängig voneinander für Wasserstoff oder $C_1$-$C_4$-Alkyl stehen oder mit dem zugehörigen Kohlenstoffatom eine

> C = O Gruppe bilden,

X und Y unabhängig voneinander die Bedeutungen Sauerstoff, Schwefel oder $NR^{10}$ besitzen, wobei $R^{10}$ Wasserstoff, $C_1$-$C_8$-Alkyl oder $C_7$-$C_{12}$-Aralkyl ist,

$R^9$ für Wasserstoff, $C_1$-$C_{22}$-Alkyl, das durch Hydroxyl, Carbonyl, $C_1$- bis $C_4$-Alkoxycarbonyl, Carboxyl, Carbamoyl, Sulfonyl, Sulfinyl oder $CH_2$-$CH_2$-SHsubstituiert sein kann, $C_3$-$C_{22}$-Alkenyl, $C_3$-$C_{22}$-Alkinyl, $C_7$-$C_{12}$-Aralkyl, $C_1$-$C_{22}$-Alkyl, $C_4$-$C_{22}$-Cycloalkylalkyl, einen Heterocyclus der aus der Gruppe

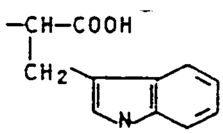 , 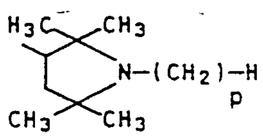 , 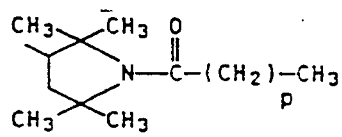 , 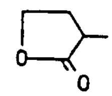

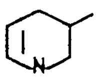 , 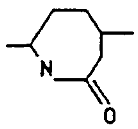 , 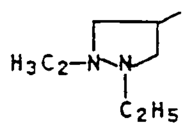 , 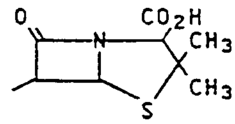 ,

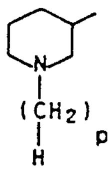 , 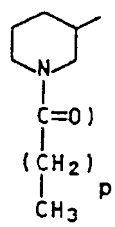 , 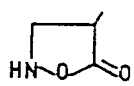 , 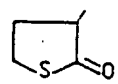 ,

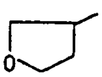 , 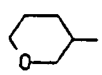 , 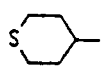 , 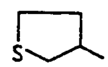 ,

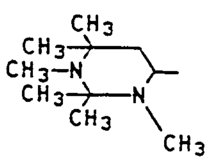 ,

wobei p = 1 bis 20 bedeutet,

Hydroxy, Carboxyl, $C_1$- bis $C_4$-Alkoxycarbonyl, Sulfonamido, $(R^{13})_2NCO$-, worin $R^{13}$ für Wasserstoff oder $C_1$-$C_4$-Alkyl steht, eine Harnstoffgruppierung der Formel

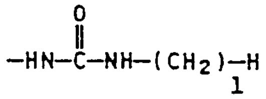 , 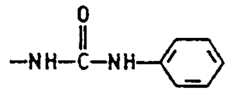 , 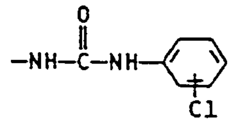 ,

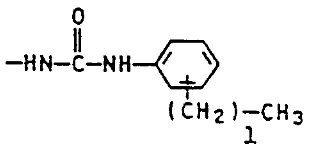 , 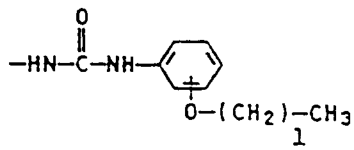 ,

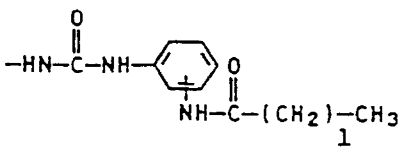 , 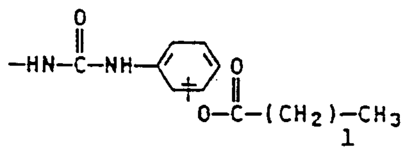 ,

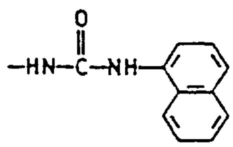 oder 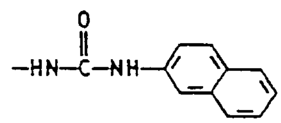 ,

mit l = 0 bis 22,
eine Urethangruppe der Formel

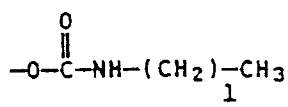

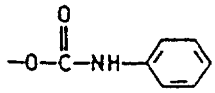 , 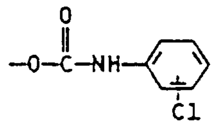 , 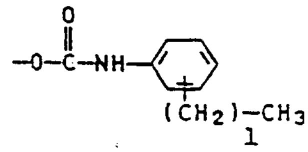

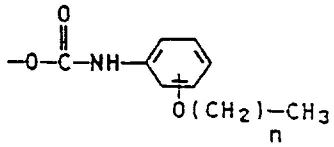 , 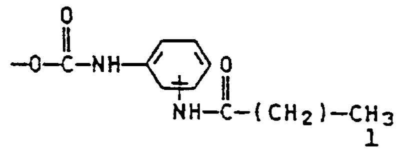 ,

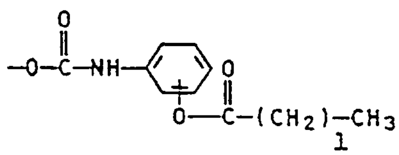 , 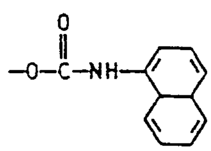 oder

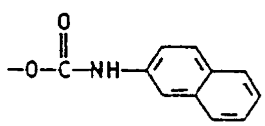 ,

mit l = 0 bis 22, oder für

$-(CH_2)_k D$

steht, worin k eine Zahl von 1 bis 10 und D -CN, $-NH_2$, $-NHR^{11}$ oder $-NR^{11}R^{12}$ bedeuten, worin $R^{11}$ und $R^{12}$ gleich oder verschieden sind und für $C_1$-$C_{22}$-Alkyl, eine Acylgruppe der Formel

$$-\overset{\overset{O}{\|}}{C}-(CH_2)_l-CH_3$$

mit l = 0 bis 22, Carbamoyl, Sulfonyl, Sulfinyl, $C_2$-$C_{22}$-Alkenyl, $C_3$-$C_{22}$-Alkinyl, $C_3$-$C_{12}$-Cycloalkyl, oligomeres oder polymeres Polyamin der Formel

$H_2N-(CH_2-CH_2-NH)_x$ (x = 1 bis 30)

stehen,
oder worin $R^{11}$ und $R^{12}$ zusammen mit dem Stickstoffatom, an das sie gebunden sind, ein 3- bis 20-gliedriges Ringsystem bilden,

A gleich oder verschieden ist und eine direkte Bindung oder ein Brückenglied der Formel

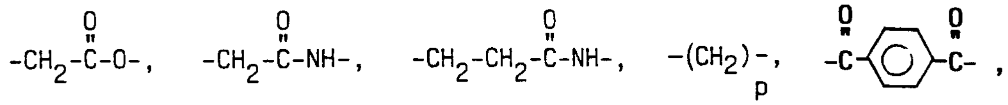

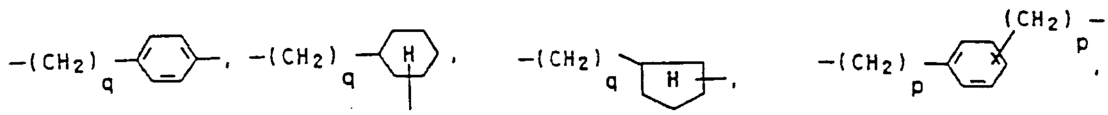

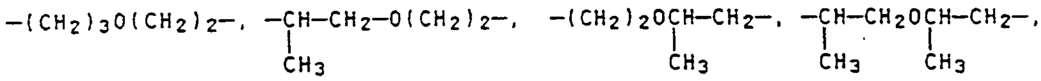

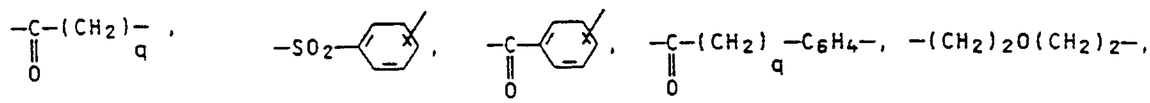

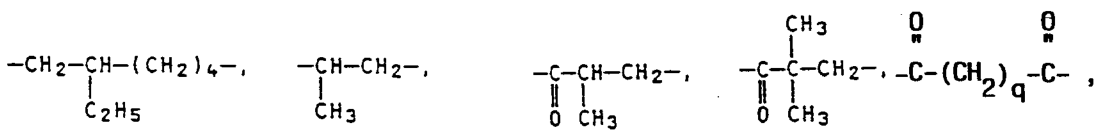

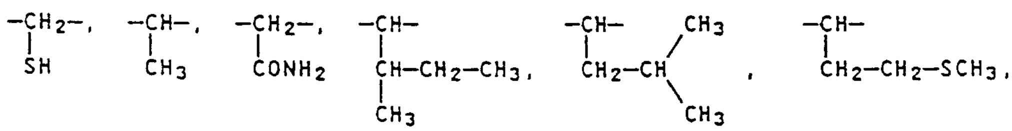

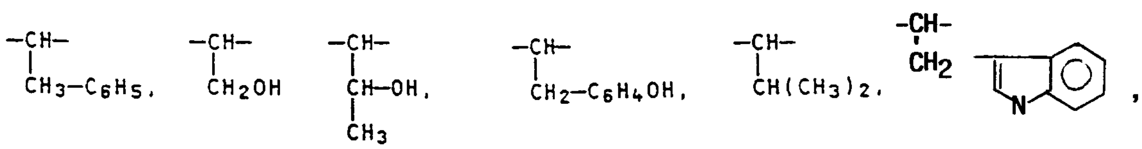

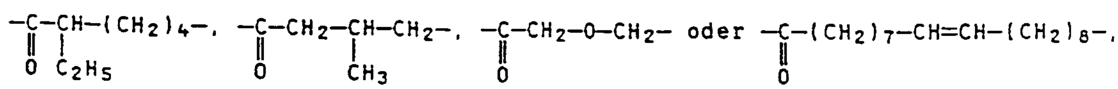

wobei p = 1 bis 20 und q bis zu 4 ist,
darstellt mit der Maßgabe, daß mindestens einer der Reste A für ein Brückenglied steht, falls $R^9$ nicht die Bedeutung $-(CH_2)_k$ D besitzt, und

B eine direkte Bindung darstellt,

und die Säureadditionssalze und Hydrate der Verbindungen der allgemeinen Formel I.

**2.** Verbindungen nach Anspruch 1, worin $R^1$ und/oder $R^2$ für Wasserstoff, Methyl, Ethyl, Benzyl, Phenyl, Carbomethoxy oder Carboethoxy stehen.

**3.** Verbindungen nach Anspruch 1, worin $R^1$ und/oder $R^2$ für Wasserstoff, Methyl oder Phenyl stehen.

**4.** Verbindungen nach Anspruch 1, 2 oder 3, worin $R^9$ für Wasserstoff steht und n = 1 ist, oder worin $R^9$ die Bedeutung $-CH_2-CN$ besitzt.

**5.** Verbindungen nach einem der Ansprüche 1 bis 4, worin $R^3$, $R^4$, $R^5$ und $R^6$ für Methyl stehen.

**6.** Verbindungen nach einem der Ansprüche 1 bis 5, worin $R^8$ für Wasserstoff steht.

**7.** Verbindungen nach einem der Ansprüche 1 bis 6, worin A und/oder B für ein Brückenglied mit einer

$$-\overset{O}{\overset{\|}{C}}-O- \text{ oder } -\overset{O}{\overset{\|}{C}}-NH\text{-Funktion}$$

stehen.

**8.** Verbindungen nach einem der Ansprüche 1 bis 7, worin X und Y für Sauerstoff stehen.

**9.** Gemische von Verbindungen der allgemeinen Formel I nach einem der Ansprüche 1 bis 8.

**10.** Verwendung der Verbindungen gemäß den Ansprüchen 1 bis 9 zum Stabilisieren von Kunststoffen und Lacken.

**11.** Verwendung gemäß Anspruch 10 zum Stabilisieren von Polyolefinen und Polyamiden.

**12.** Verwendung nach einem der Ansprüche 10 oder 11 zur Licht- und Wärmestabilisierung oder als Metallionendesaktivator.

**13.** Stabilisierte Kunststoffe oder Lacke, enthaltend mindestens eine Verbindung der allgemeinen Formel I nach einem der Ansprüche 1 bis 9.

**14.** Produkt, erhalten durch die Umsetzung des Amins folgender Struktur

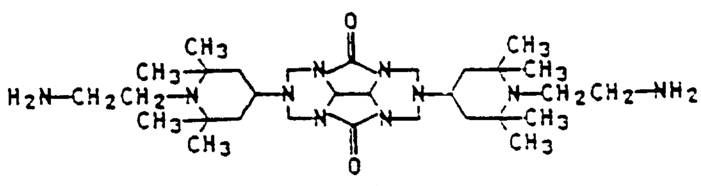

mit Adipinsäurechlorid im Molverhältnis 1:1 in Pyridin, Fällung durch Petrolether und Aufarbeitung mit 10% NaOH, welches folgendes Strukturelement enthält

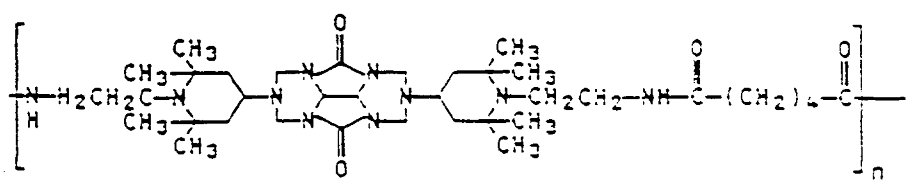

vom Schmelzpunkt 274 °C und einer mittleren Molmasse von 2570 g/mol (dampfdruckosmometrisch in Chloroform).

## Claims

**1.** A compound of the formula (I)

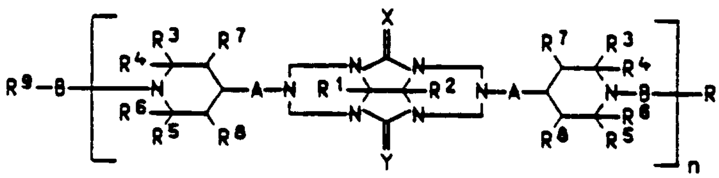

(I)

where

n is 1

$R^1$ and $R^2$ are independently of each other hydrogen, $C_1$-$C_6$-alkyl, $C_7$-$C_{12}$-aralkyl, unsubstituted or $C_1$-$C_4$-alkyl-, $C_1$-$C_4$-alkoxy- or halogen-substituted phenyl or naphthyl or a carboxylic ester of the formula

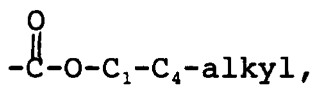

or $R^1$ and $R^2$ together are a tetra-, penta- or hexamethylene group or a radical of the formula

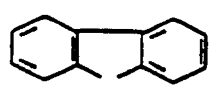

$R^3$, $R^4$, $R^5$ and $R^6$ are independently of each other $C_1$-$C_6$-alkyl,

$R^7$ and $R^8$ are independently of each other hydrogen or $C_1$-$C_4$-alkyl or together with the associated carbon atom form a $>C=O$ group,

X and Y independently of each other have the meanings oxygen, sulfur or $NR^{10}$, where $R^{10}$ is hydrogen, $C_1$-$C_8$-alkyl or $C_7$-$C_{12}$-aralkyl,

$R^9$ is hydrogen, $C_1$-$C_{22}$-alkyl which may be substituted by hydroxyl, carbonyl, $C_1$-$C_4$-alkoxycarbonyl, carboxyl, carbamoyl, sulfonyl, sulfinyl or $CH_2$-$CH_2$-SH, $C_3$-$C_{22}$-alkenyl, $C_3$-$C_{22}$-alkynyl, $C_7$-$C_{22}$-aralkyl, $C_1$-$C_{22}$-alkyl, $C_4$-$C_{22}$-cycloalkylalkyl, a heterocycle from the group

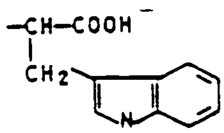 , 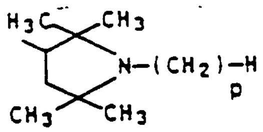 , 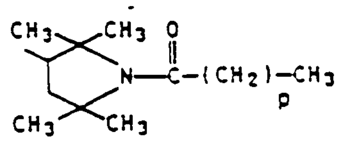 , 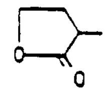

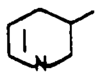 , 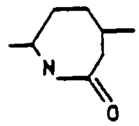 , 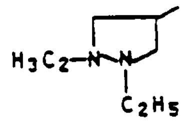 , 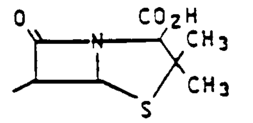 ,

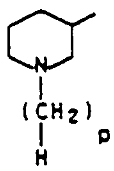 , 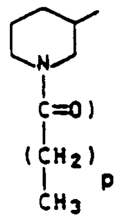 , 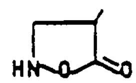 , 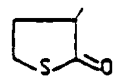 ,

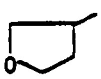 , 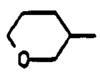 , 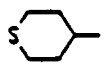 , 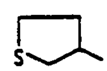 ,

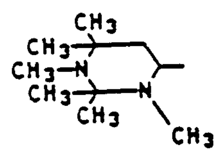 ,

where p is from 1 to 20,
hydroxyl, carboxyl, $C_1$-$C_4$-alkoxycarbonyl, sulfonamido, $(R^{13})_2NCO$-, where $R^{13}$ is hydrogen or $C_1$-$C_4$-alkyl, a urea group of the formula

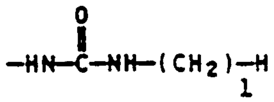 , 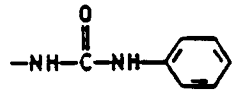 , 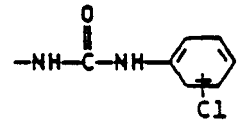 ,

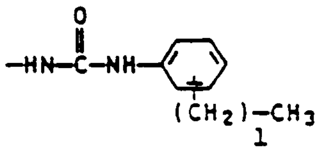 , 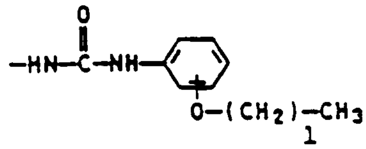 ,

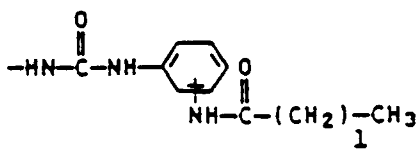 , 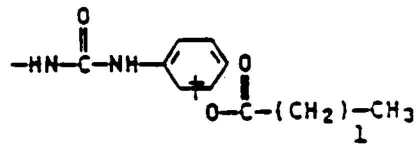 ,

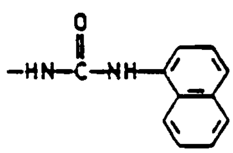 or 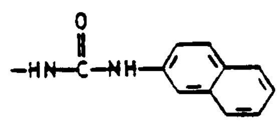 ,

a urethane group of the formula where l is from 0 to 22

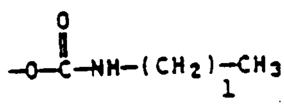

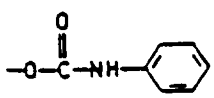 , 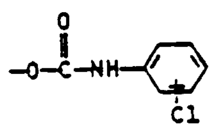 , 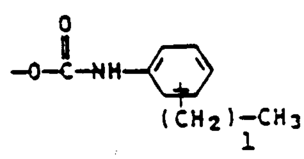

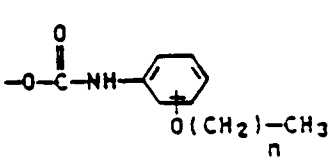 , 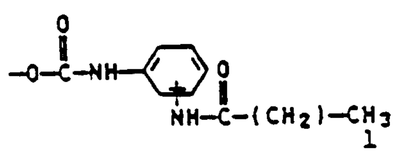 ,

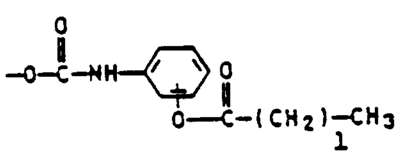 , 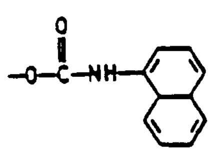 or

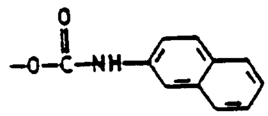 .

where l is from 0 to 22 or

$-(CH_2)_k D$

where k is from 1 to 10 and D is -CN, $-NH_2$, $-NHR^{11}$ or $-NR^{11}R^{12}$, where $R^{11}$ and $R^{12}$ are identical or different and each is $C_1$-$C_{22}$-alkyl, an acyl group of the formula

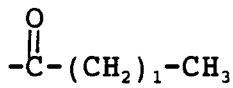,

where l is from 0 to 22, carbamoyl, sulfonyl, sulfinyl, $C_2$-$C_{22}$-alkenyl, $C_3$-$C_{22}$-alkynyl, $C_3$-$C_{12}$-cycloalkyl or oligomeric or polymeric polyamine of the formula

$H_2N\{CH_2-CH_2-NH\}_x$ (x = from 1 to 30)

or where $R^{11}$ and $R^{12}$ together with the nitrogen atom to which they are bonded are a 3- to 20-membered ring system, the

A's are identical or different and each is a direct bond or a bridge member of the formula

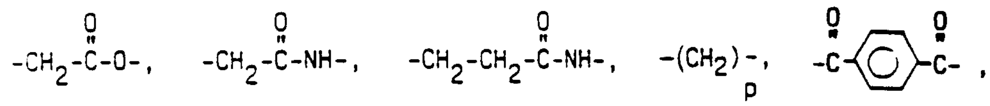

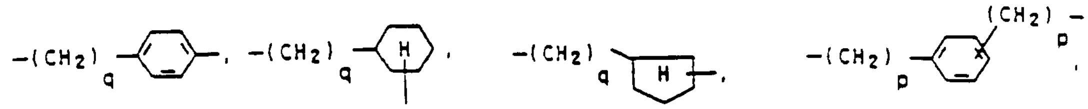

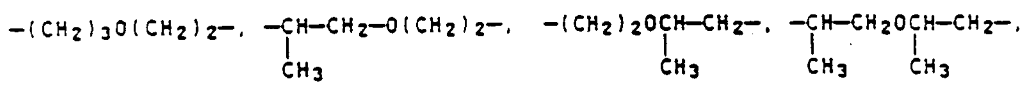

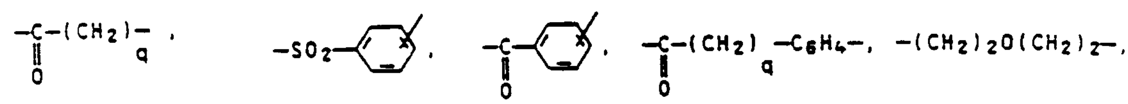

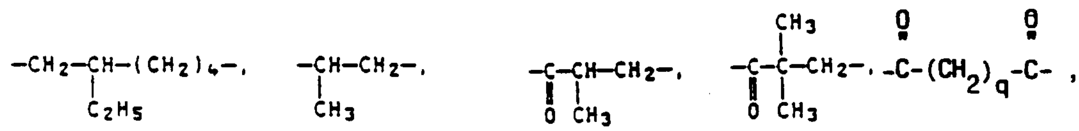

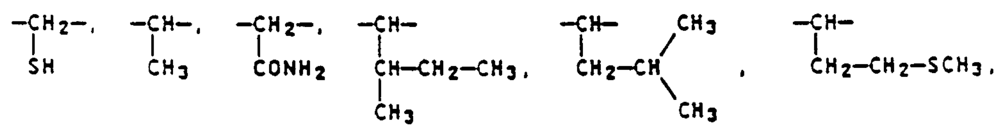

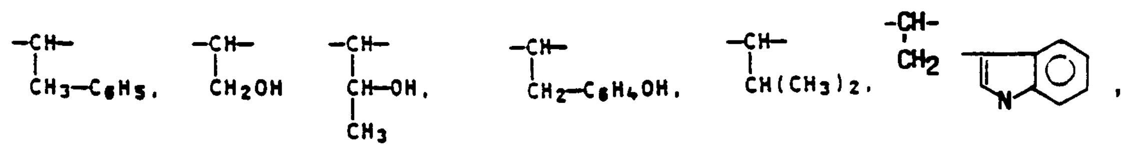

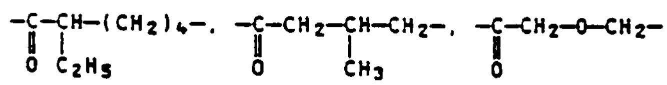

oder

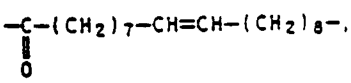

where p is from 1 to 20 and q is up to 4, with the proviso that one or more of the radicals A is a bridge member if $R^9$ does not have the meaning $-(CH_2)_k-D$, and

B is a direct bond,

and the acid addition salts and hydrates thereof.

2. A compound as claimed in claim 1, where $R^1$ and/or $R^2$ are each hydrogen, methyl, ethyl, benzyl, phenyl, carbomethoxy or carboethoxy.

3. A compound as claimed in claim 1, where $R^1$ and/or $R^2$ are each hydrogen, methyl or phenyl.

4. A compound as claimed in claim 1 or 2 or 3, where $R^9$ is hydrogen and n is 1, or where $R^9$ is $-CH_2-CN$.

5. A compound as claimed in claim 1 or 2 or 3 or 4, where $R^3$, $R^4$, $R^5$ and $R^6$ are each methyl.

6. A compound as claimed in claim 1 or 2 or 3 or 4 or 5, where $R^8$ is hydrogen.

7. A compound as claimed in claim 1 or 2 or 3 or 4 or 5 or 6, where A and/or B are each a bridge member having a

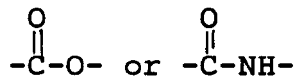

function.

8. A compound as claimed in claim 1 or 2 or 3 or 4 or 5 or 6 or 7, where X and Y are each oxygen.

9. A mixture of compounds of the formula I as claimed in claim 1 or 2 or 3 or 4 or 5 or 6 or 7 or 8.

10. Use of a compound as claimed in claim 1 or 2 or 3 or 4 or 5 or 6 or 7 or 8 or 9 for stabilizing plastics and paints.

11. Use as claimed in claim 10 for stabilizing polyolefins and polyamides.

12. Use as claimed in claim 10 or 11, for light and heat stabilization or as a metal ion deactivator.

13. A stabilized plastic or paint comprising one or more compounds of the formula I as claimed in claim 1 or 2 or 3 or 4 or 5 or 6 or 7 or 8 or 9.

14. A product obtained by reacting the amine of the following structure:

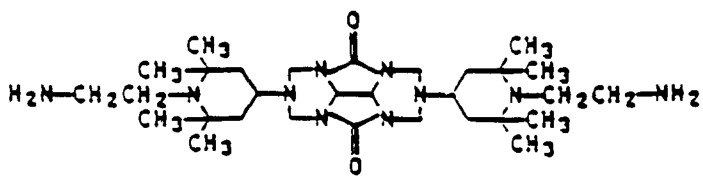

with adipoyl chloride in a molar ratio of 1:1 in pyridine, precipitating with petroleum ether and working up with 10 % strength NaOH, containing the following structural element:

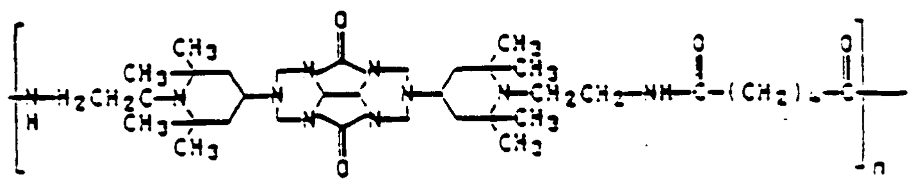

and having a melting point of 274 °C and an average molecular weight of 2570 g/mol (by vapor pressure osmometry in chloroform).

## Revendications

1. Composés de la formule générale (I)

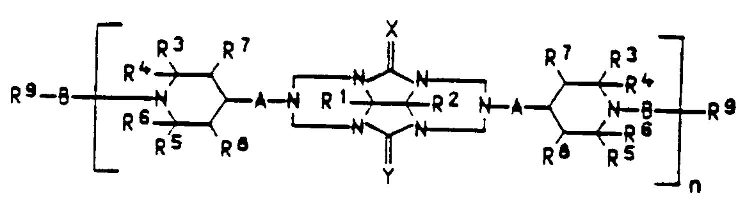 (I),

dans laquelle

n — est égal à 1,

$R^1$ et $R^2$, — représentent, indépendamment l'un de l'autre, des atomes d'hydrogène, des radicaux alkyle en $C_1$-$C_6$, aralkyle en $C_7$-$C_{12}$, naphtyle, ou phényle éventuellement substitué par des radicaux alkyle en $C_1$-$C_4$, alcoxy en $C_1$-$C_4$ ou des atomes d'halogènes, ou un radical ester carboxylique de la formule

$$-\overset{\overset{\displaystyle O}{\|}}{C}-O\text{-alkyle}$$

en $C_1$-$C_4$, ou bien $R^1$ et $R^2$ forment ensemble un groupe tétra-, penta-, ou hexaméthylène, ou un radical de la formule

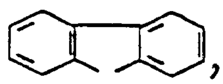

$R^3$, $R^4$, $R^5$ et $R^6$ — représentent, indépendamment l'un de l'autre, des radicaux alkyle en $C_1$-$C_6$,

$R^7$ et $R^8$ — représentent, indépendamment l'un de l'autre, des atomes d'hydrogène ou des radicaux alkyle en $C_1$-$C_4$, ou forment avec l'atome de carbone concerné, un groupe
$>C=O$

X et Y — représentent, indépendamment l'un de l'autre, des atomes d'oxygène, de soufre ou des radicaux $NR^{10}$ où $R^{10}$ représente un atome d'hydrogène, un radical alkyle en $C_1$-$C_8$ ou aralkyle en $C_7$-$C_{12}$,

$R^9$ — représente un atome d'hydrogène, un radical alkyle en $C_1$-$C_{22}$ qui peut être substitué par des radicaux hydroxyle, carbonyle, alcoxy($C_1$-$C_4$)carbonyle, carboxyle, carbamoyle, sulfonyle, sulfinyle, ou $CH_2$-$CH_2$-SH, un radical alcényle en $C_3$-$C_{22}$, alcynyle en $C_3$-$C_{22}$, aralkyle en $C_7$-$C_{12}$, alkyle en $C_1$-$C_{22}$, cycloalkylalkyle en $C_4$-$C_{22}$, un hétérocycle appartenant au groupe

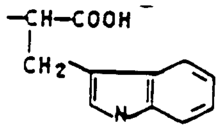 , 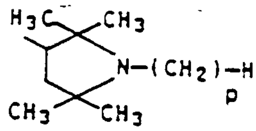 , 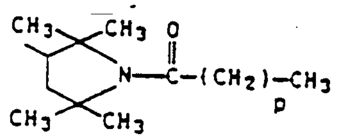 , 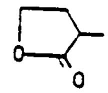

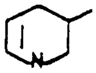 , 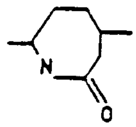 , 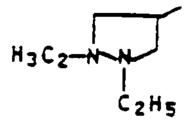 , 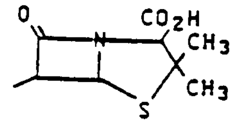 ,

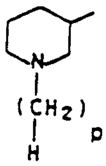 , 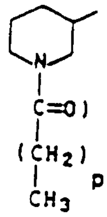 , 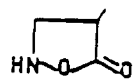 , 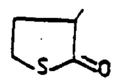 ,

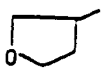 , 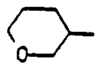 , 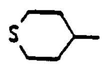 , 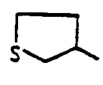 ,

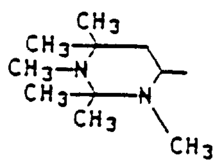 ,

où p varie de 1 à 20,

hydroxyle, carboxyle, alcoxy($C_1$-$C_4$)carbonyle, sulfonamido, $(R^{13})_2NCO$-, où $R^{13}$ représente un atome d'hydrogène ou un radical alkyle en $C_1$-$C_4$, un groupement urée de la formule

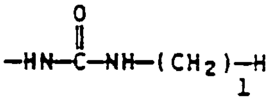 , 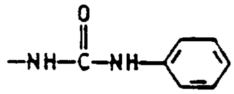 , 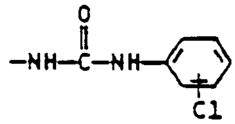 ,

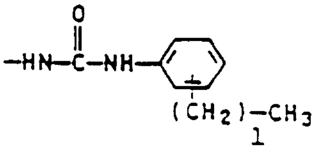 , 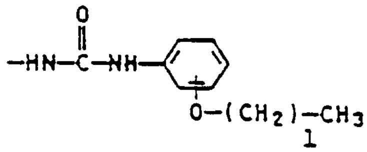 ,

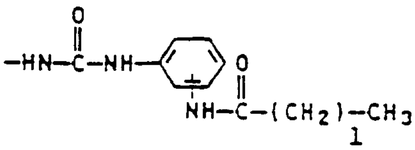 , 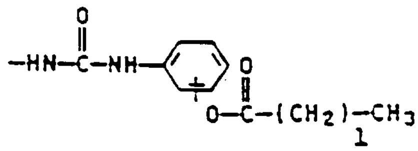 ,

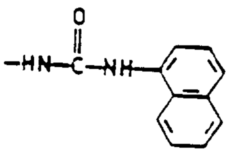 ou 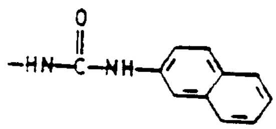 ,

avec 1 = 0 à 22, un radical uréthanne de la formule

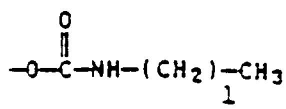

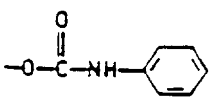 , 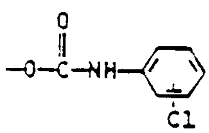 , 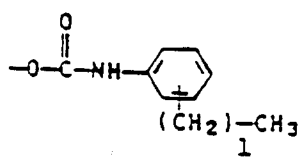

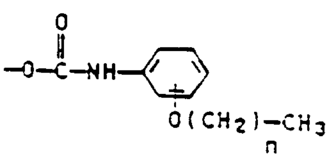 , 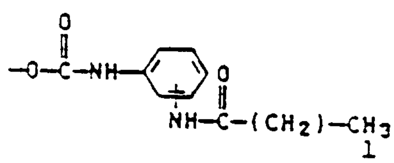 ,

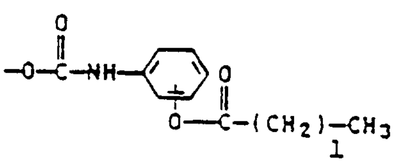 , 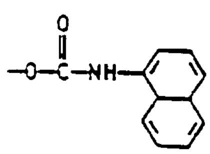 ou

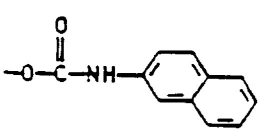 ,

avec 1 = 0 à 22,
ou

$-(CH_2)_kD$

où k représente un nombre dont la valeur varie de 1 à 10 et D représente un radical -CN, $-NH_2$, $-NHR^{11}$ ou $-NR^{11}R^{12}$, où $R^{11}$ et $R^{12}$ peuvent être identiques ou différents et représentent des radicaux alkyle en $C_1$-$C_{22}$, acyle de la formule

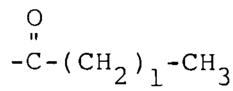

avec l = 0 à 22, carbamoyle, sulfonyle, sulfinyle, alcényle en $C_2$-$C_{22}$, alcynyle en $C_3$-$C_{22}$, cycloalkyle en $C_3$-$C_{12}$, une polyamine oligomérique ou polymérique de la formule

$H_2N\{CH_2-CH_2-NH\}_x$ (x = 1 bis 30)

ou bien où $R^{11}$ et $R^{12}$ forment un système cyclique à 3-20 chaînons avec l'atome d'azote auquel ils sont attachés,

A identique ou différent, représente une liaison directe, ou un élément de pontage de la formule

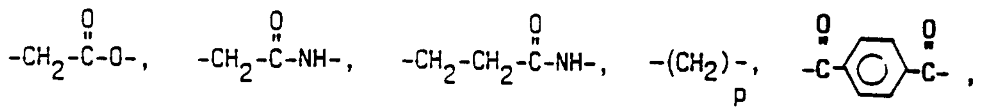

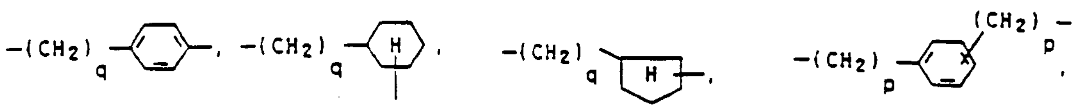

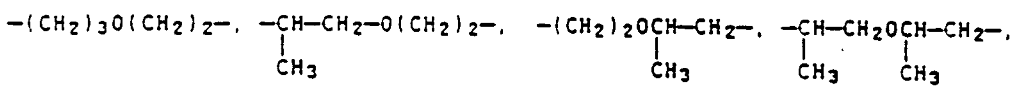

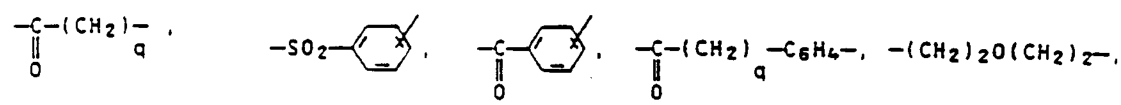

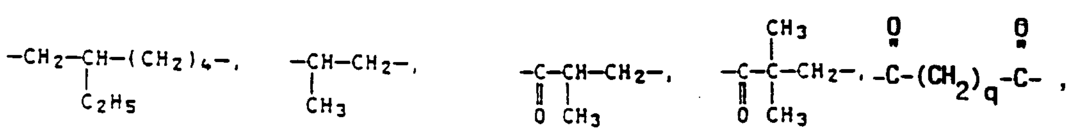

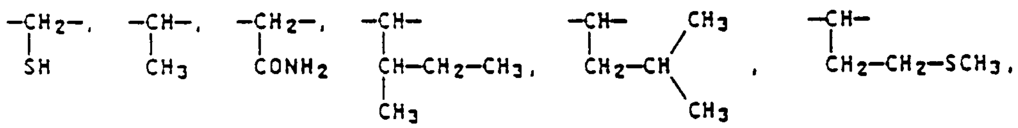

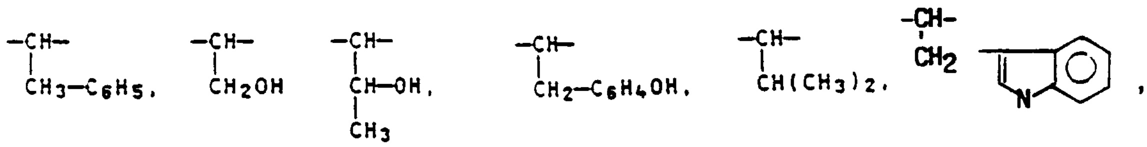

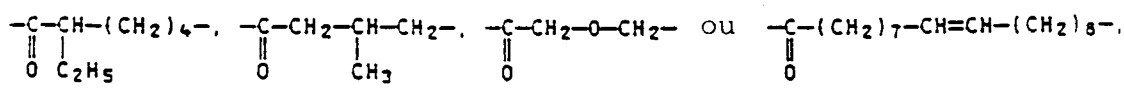

où p = 1 à 20 et q s'élève jusqu'à 4,
avec la condition qu'au moins l'un des restes A représente un élément de pontage, au cas où $R^9$ n'a pas la signification $-(CH_2)_k$ D, et
B représente une liaison directe,
ainsi que les sels d'addition d'acides et les hydrates des composés de la formule générale I.

**2.** Composés suivant la revendication 1, caractérisés en ce que $R^1$ et/ou $R^2$ représentent des atomes d'hydrogène, des radicaux méthyle, éthyle, benzyle, phényle, carbométhoxy, ou carbéthoxy.

**3.** Composés suivant la revendication 1, caractérisés en ce que $R^1$ et/ou $R^2$ représentent des atomes d'hydrogène, des radicaux méthyle ou phényle.

**4.** Composés suivant la revendication 1, 2 ou 3, caractérisés en ce que $R^9$ représente un atome d'hydrogène et n est égal à 1, ou en ce que $R^9$ désigne un groupe $-CH_2-CN$.

**5.** Composés suivant l'une quelconque des revendications 1 à 4, caractérisés en ce que $R^3$, $R^4$, $R^5$ et $R^6$ représentent des radicaux méthyle.

**6.** Composés suivant l'une quelconque des revendications 1 à 5, caractérisés en ce que $R^8$ représente un atome d'hydrogène.

**7.** Composés suivant l'une quelconque des revendications 1 à 6, caractérisés en ce que A et/ou B représentent un élément de pontage avec une fonction

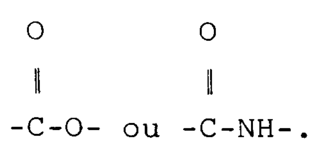

**8.** Composés suivant l'une quelconque des revendications 1 à 7, caractérisés en ce que X et Y représentent des atomes d'oxygène.

**9.** Mélanges de composés de la formule générale I suivant l'une quelconque des revendications 1 à 8.

**10.** Utilisation des composés suivant l'une quelconque des revendications 1 à 9 pour la stabilisation de matières plastiques et de laques ou peintures.

**11.** Utilisation suivant la revendication 10 en vue de la stabilisation de polyoléfines et de polyamides.

**12.** Utilisation suivant l'une quelconque des revendications 10 et 11 pour la stabilisation vis-à-vis de la lumière ou de la chaleur, ou à titre de désactivateur d'ions de métaux.

**13.** Peintures ou laques, ou matières plastiques, stabilisées, contenant au moins un composé de la formule générale I suivant l'une quelconque des revendications 1 à 9.

**14.** Produit obtenu par la réaction de l'amine de la structure suivante

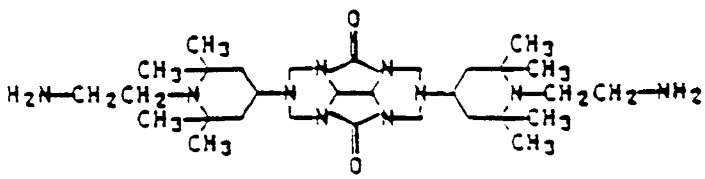

avec le chlorure d'acide adipique, dans le rapport molaire de 1:1, dans la pyridine, précipitation par l'éther de pétrole et traitement avec du NaOH à 10%, qui contient un élément de structure suivant

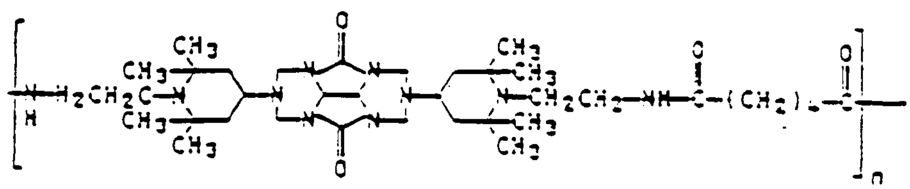

d'un point de fusion de 274 °C et d'une masse molaire moyenne de 2570 g/mole (telle que mesurée par voie osmométrique à pression de vapeur dans le chloroforme).